# EUROPEAN PATENT SPECIFICATION

(11) **EP 1 007 121 B1**
(45) Date of publication and mention of the grant of the patent: **11.06.2003**
(21) Application number: 98909948.6
(22) Date of filing: 13.03.1998
(51) Int. Cl.: A61M 15/00

(54) **INHALATION DEVICE**
INHALATIONSGERÄT
INHALATEUR

(30) Priority: 14.03.1997 SE 9700939
(43) Date of publication of application: 14.06.2000
(73) Proprietor: AstraZeneca AB, 151 85 Södertälje (SE)
(72) Inventor: DAGSLAND, Allan, S-374 40 Karlshamn (SE); VIRTANEN, Risto, FIN-01900 Nurmijärvi (FI)
(86) International application number: SE9800459
(87) International publication number: WO98041258

(56) References cited:
- WO-A-86/05991
- WO-A-94/14492

## Description

The present invention relates to a powder inhaler for administering powder by inhalation.

A number of powder inhalers are known which use different systems for introducing a dose of powder into an air stream. Typically, the powder is inhaled into the lungs of a patient in order to treat, for example, asthma.

One such powder inhaler is disclosed in WO-A-86/05991. This inhaler includes a flow path, which comprises an inhalation channel and a mouthpiece comprising an air chamber and an outlet nozzle, through which a stream of air is drawn during inhalation by a user, and means for introducing powder into the inhalation channel. During inhalation, air is first drawn into and through the inhalation channel so as to pick up powder. The stream of air containing powder is then drawn through the air chamber and out of the outlet nozzle of the mouthpiece.

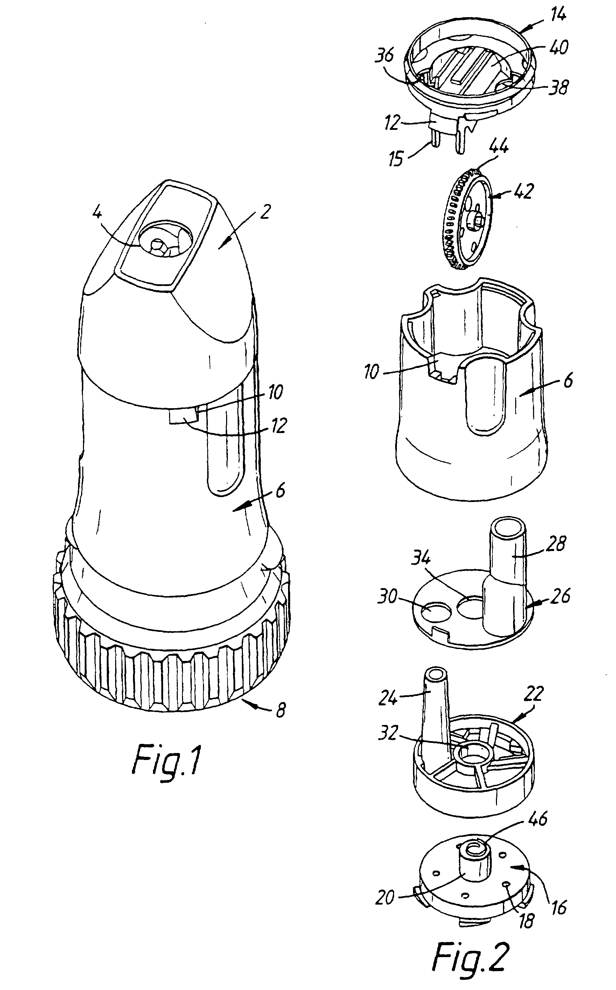

Figure 1 illustrates such a powder inhaler. The inhaler comprises a mouthpiece 2 comprising an air chamber (not illustrated) and an outlet nozzle 4, an inhaler body 6 and a rotatable grip portion 8 for operating a dosing mechanism for providing doses of powder for inhalation. The inhaler body 6 includes an opening 10 which is filled with a window 12 through which an indicating wheel 42 is visible so as to provide an indication as to the usage of the inhaler.

Figure 2 illustrates in exploded view component parts disposed within and to the inhaler body 6. The inhaler body 6 is capped with a divider 14 which is fixed thereto and separates the air chamber in the mouthpiece 2 from a major part of the inhaler body 6. For aesthetic reasons the inhaler body 6 is an opaque moulding. The divider 14 is a transparent moulding which has a depending tongue 15, a part of which forms the window 12. When the inhaler is assembled, the only part of the divider 14 which is visible is the part of the tongue 15 forming the window 12, and thus the overall appearance of the inhaler is unaffected.

Within the inhaler body 6 are housed the component parts of the dosing mechanism. These component parts include a dosing unit 16 which comprises a plurality of dosing means 18 and a central axial shaft 20, an inhalation unit 22 which comprises an inhalation channel 24 and a storage unit 26 which comprises a storage chamber 28 for storing powder. The above-mentioned component parts of the dosing mechanism are assembled by passing the inhalation channel 24 through an opening 30 in the storage unit 26 and passing the shaft 20 through central openings 32, 34 in the inhalation unit 22 and the storage unit 26 respectively. When so assembled, the upper ends of the inhalation channel 24 and the storage chamber 28 pass respectively through first and second openings 36, 38 in the divider 14.

In use, powder is transferred from the storage chamber 28 to one of the dosing means 18, and, with rotation of the dosing unit 16, the one dosing means 18 provides a dose of powder to the inhalation channel 24. On inhalation by a user the powder is drawn up through the air chamber and out of the outlet nozzle 4 of the mouthpiece 2.

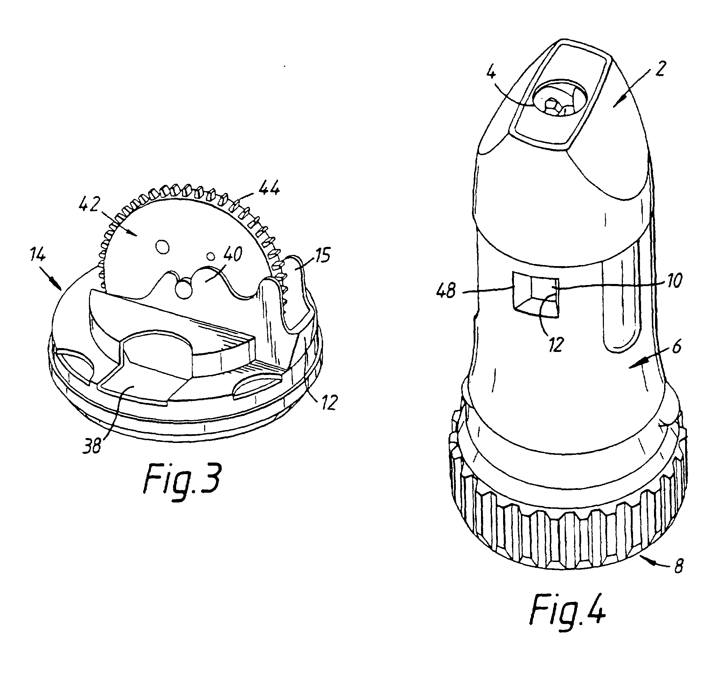

As illustrated in Figures 2 and 3, the divider 14 further comprises supporting means 40 for rotatably supporting an indicating wheel 42. The indicating wheel 42 has a plurality of teeth 44 disposed around the periphery thereof which engage with a spiral groove or protrusion 46 on the end face of the shaft 20 of the dosing unit 16. The supporting means 40 is configured to align the indicating wheel 42 such that a part of the periphery thereof is disposed adjacent the inner surface of the window 12.

In use, as the dosing unit 16 is rotated, the spiral groove or protrusion 46 engages with one or more of the teeth 44 on the indicating wheel 42 so as to rotate the same. In this way, by providing a coloured marking on the periphery of the indicating wheel 42, it is possible to provide the user with a visible indication at the window 12 as to the usage of the inhaler.

Whilst the above-described known powder inhaler functions quite adequately, it is an aim of the present invention to provide a powder inhaler which more visibly indicates the usage of the inhaler. It is a further aim of the present invention to provide a powder inhaler having fewer components and hence reduced manufacturing complexity.

Accordingly, the present invention provides a powder inhaler, comprising: a substantially cylindrical inhaler body having an opening therein; an inhalation unit disposed in the inhaler body, the inhalation unit comprising an inhalation channel through which powder is in use inhaled; a dosing unit for providing a dose of powder to the inhalation channel disposed in the inhaler body so as to be rotatable about the central axis thereof, wherein the dosing unit comprises a central shaft which is co-axial with the central axis of the inhaler body and has a spiral groove or protrusion on the end face thereof; and an indicating wheel for providing an indication as to the usage of the inhaler disposed in the inhaler body, the indicating wheel having a toothed periphery for engaging the spiral groove or protrusion on the shaft and being disposed such that at least a part thereof is visible through the opening and so as to be rotatable within a diametrical plane containing the central axis of the inhaler body; characterized in that one side surface of the indicating wheel includes at least one indication which is representative of the usage of the inhaler, in that the inhaler body includes a recess in which the opening is provided and in that the opening has a radial component which allows at least a part of the one side surface of the indicating wheel to be viewed.

Preferably, the inhaler further comprises a storage unit disposed in the inhaler body, the storage unit comprising a storage chamber for storing powder.

More preferably, the storage unit is formed of a transparent material and the inhaler further comprises a portion which substantially fills the opening.

Preferably, the inhalation unit and the storage unit are formed as a single integral unit.

Preferably, the inhaler further comprises a divider substantially closing one end of the inhaler body.

More preferably, the recess comprises first and second opposing surfaces which are substantially parallel to the major surface of the divider and at least first and second side surfaces joining the first and second opposing surfaces, the opening being formed in one of the side surfaces.

Preferably, the inhaler body and the divider are formed as a single integral unit.

In one embodiment the storage unit further comprises supporting means for rotatably supporting the indicating wheel.

In another embodiment the divider comprises supporting means for rotatably supporting the indicating wheel.

Preferably, the inhaler body further comprises an air inlet in a side wall thereof, the air inlet allowing air to be drawn to the dosing unit and through the inhalation channel.

More preferably, the air inlet is provided in the recess.

Preferably, the one side surface of the indicating wheel includes numerals indicating the number of doses administered and/or remaining.

Preferably, the one side surface of the indicating wheel includes an at least part circular band of varying width indicating the number of doses administered and/or remaining.

Medicaments suitable for administration by the powder inhaler of the present invention are any which may be delivered by inhalation and include for example β2-adrenoreceptor agonists, for example, salbutamol, terbutaline, rimiterol, fenoterol, reproterol, adrenaline, pirbuterol, isoprenaline, orciprenaline, bitolterol, salmeterol, formoterol, clenbuterol, procaterol, broxaterol, picumeterol, TA-2005, mabuterol and the like, and their pharmacologically acceptable esters and salts; anticholinergic bronchodilators, for example, ipratropium bromide and the like; glucocorticosteroids, for example, beclomethasone, fluticasone, budesonide, tipredane, dexamethasone, betamethasone, fluocinolone, triamcinolone acetonide, mometasone and the like, and their pharmacologically acceptable esters and salts; antiallergic medicaments, for example, sodium cromoglycate and nedocromil sodium; expectorants; mucolytics; antihistamines; cyclooxygenase inhibitors: leukotriene synthesis inhibitors; leukotriene antagonists; phospholipase-A2 (PLA2) inhibitors; platelet aggregating factor (PAF) antagonists and prophylactics of asthma; antiarrhythmic medicaments; tranquilisers; cardiac glycosides; hormones; antihypertensive medicaments; antidiabetic medicaments; antiparasitic medicaments; anticancer medicaments; sedatives; analgesic medicaments; antibiotics; antirheumatic medicaments; immunotherapies; antifungal medicaments; antihypotension medicaments; vaccines; antiviral medicaments; proteins; polypeptides and peptides, for example, peptide hormones and growth factors; polypeptide vaccines; enzymes; endorphines; lipoproteins and polypeptides involved in the blood coagulation cascade; vitamins; and others, for example, cell surface receptor blockers, antioxidants, free radical scavengers and organic salts of N,N'-diacetylcystine.

Preferred embodiments of the present invention will now be described hereinbelow by way of example only with reference to the accompany drawings, in which:
Figure 1 illustrates a perspective view of a known powder inhaler;
Figure 2 illustrates in exploded view component parts of the inhaler of Figure 1;
Figure 3 illustrates component parts of the inhaler of Figure 1;
Figure 4 illustrates a perspective view of a powder inhaler in accordance with a first embodiment of the present invention;
Figures 5 and 6 illustrate indicating wheels for use with the powder inhaler of Figure 4;
Figure 7 illustrates in exploded view component parts of a powder inhaler in accordance with a second embodiment of the present invention;
Figure 8 illustrates in exploded view component parts of a powder inhaler in accordance with a third embodiment of the present invention;
Figures 9 and 10 illustrate component parts of a powder inhaler in accordance with a fourth embodiment of the present invention;
Figure 11 illustrates a component part of a powder inhaler in accordance with a fifth embodiment of the present invention;
Figures 12 and 13 illustrate component parts of a powder inhaler in accordance with a sixth embodiment of the present invention; and
Figures 14 and 15 illustrate component parts of a powder inhaler in accordance with a seventh embodiment of the present invention.

Structurally, the powder inhalers in accordance with the preferred embodiments of the present invention have many features in common with the above-described known powder inhaler. For this reason, and in order to avoid unnecessary duplication of description, only the structural differences will be described in detail and reference is made to the preceding description of the known powder inhaler.

Figure 4 illustrates a powder inhaler in accordance with a first embodiment of the present invention.

This inhaler is a modification of the above-described known powder inhaler. This inhaler differs from the above-described known powder inhaler in that the inhaler body 6 includes a recess 48 in a side surface of which is provided the opening 10, which recess 48 is located such that the opening 10 is adjacent one of the side surfaces of the indicating wheel 42, and in that the side surface of the indicating wheel 42 adjacent the opening 10 includes an indication or indications representative of the usage of the inhaler. As in the above-described known powder inhaler, the indicating wheel 42 is rotatably supported to the underside of the divider 14. In a preferred embodiment one or both of the inhalation unit 22 and the storage unit 26 are formed together with the inhaler body 6 as a single integral unit.

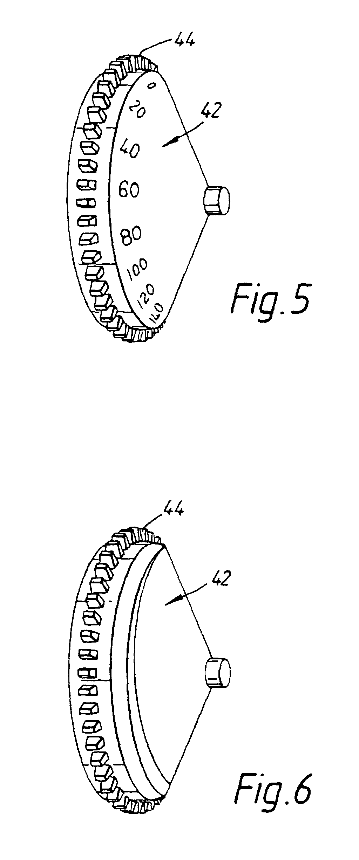

In one embodiment the indicating wheel 42 can be provided with numeric indications of increasing or decreasing value, for indicating the number of times the inhaler has been operated or the number of times the inhaler may still be operated. In another embodiment the indicating wheel 42 may alternatively, or additionally, be provided with a circumferential band of changing width along its length, such that the width visible through the window 12 is representative of the number of doses delivered. Colour changes may also be used to indicate the number of doses delivered or remaining. Such colour changes may be applied in conjunction with the indications described hereinabove. For instance, by using numerals of different colour, or by using a band, the colour of which changes along its length. In a preferred embodiment, in order to assist viewing, the side surface of the indicating wheel 42 adjacent the window 12 can be formed as a conical surface, with the surface of the cone enclosing an angle of from 10° to 30°, preferably about 15°, with the rotational plane of the indicating wheel 42. Figures 5 and 6 illustrate preferred indicating wheels 42.

In this embodiment the recess 48 is configured such that the side surface thereof in which the opening 10 is provided is parallel to the adjacent side surface of the indicating wheel 42. It will be appreciated, however, that, for the purposes of viewing the indicating wheel 42, it is sufficient that the opening 10 has a radial component. It will also be appreciated that the recess 48 can have any shape which allows a user to view the adjacent side surface of the indicating wheel 42 through the opening 10.

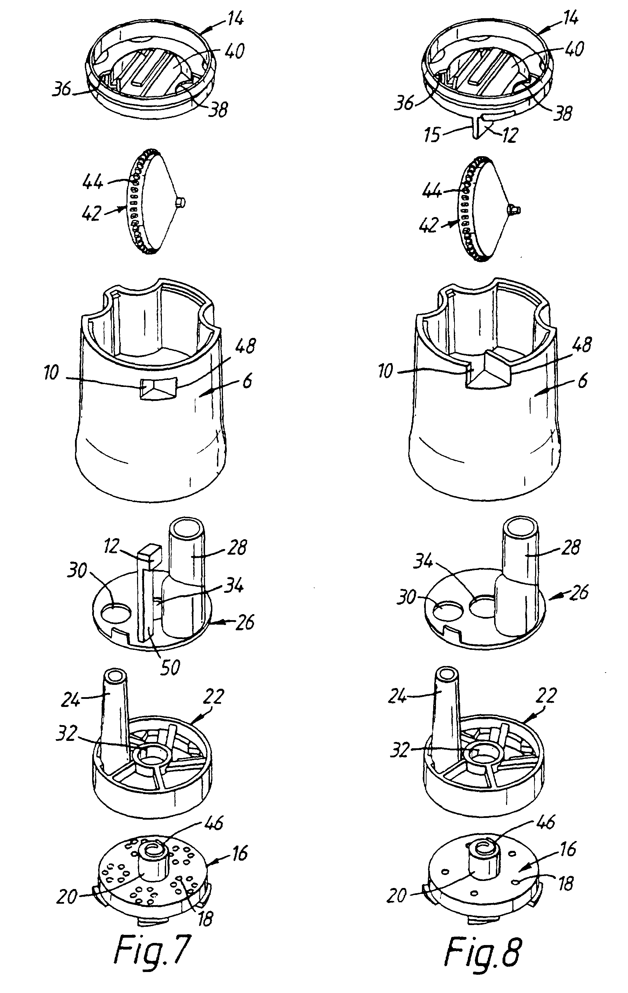

Figure 7 illustrates in exploded view component parts disposed within and to the inhaler body 6 of a powder inhaler in accordance with a second embodiment or the present invention.

This inhaler is a modification of the inhaler of the above-described first embodiment. This inhaler differs from the inhaler of the above-described first embodiment in that the recess 48 in the inhaler body 6 is of different shape. In this embodiment the recess 48 is of triangular cross-section. It will be noted, however, that in common with the inhaler of the above-described first embodiment the opening 10 in the recess 48 has a radial component. This inhaler further differs from the inhaler of the above-described first embodiment in that the divider 14 does not include a depending tongue 15, but rather the storage unit 26 is formed from a transparent material and comprises an upstanding tongue 50, one part, in this embodiment the distal end, of which is shaped and dimensioned so as to provide the window 12 and fill the opening 10 when the storage unit 26 is fitted in the inhaler body 6.

Figure 8 illustrates in exploded view component parts disposed within and to the inhaler body 6 of a powder inhaler in accordance with a third embodiment of the present invention.

This inhaler is a modification of the inhaler of the above-described first embodiment. This inhaler differs from the inhaler of the above-described first embodiment in that the recess 48 in the inhaler body 6 is of different shape. In this embodiment the recess 48 is, similarly to the recess 48 of the above-described second embodiment, of triangular cross-section. It will be noted, however, that again in common with the inhaler body 6 of the above-described first embodiment the opening 10 in the recess 48 has a radial component. This inhaler further differs from the above-described first embodiment in that the tongue 15 depending from the divider 14 is oriented substantially radially as opposed to circularly so as to align with the opening 10 in the recess 48. Again, as in the above-described known powder inhaler, the indicating wheel 42 is rotatably supported to the underside of the divider 14.

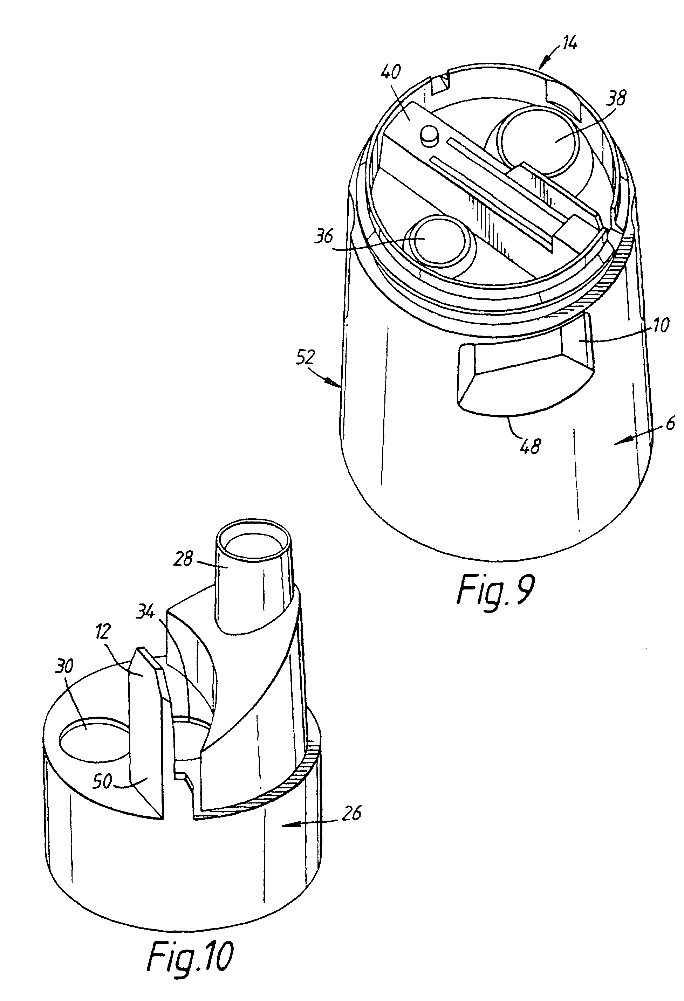

Figures 9 and 10 illustrate respectively a body part 52 and a storage unit 26 of a powder inhaler in accordance with a fourth embodiment of the present invention.

This inhaler is a modification of the inhaler of the above-described first embodiment. This inhaler differs from the inhaler of the above-described first embodiment in comprising a body part 52 which is a single part moulded from an opaque material that comprises both the inhaler body 6 and the divider 14. This inhaler further differs from the inhaler of the above-described first embodiment in that the divider 14 does not include a depending tongue 15, but rather the storage unit 26 is formed from a transparent material and comprises an upstanding tongue 50, one part, in this embodiment the distal end, of which is shaped and dimensioned so as to provide the window 12 and fill the opening 10 when the storage unit 26 is fitted in the inhaler body 6. As in the above-described known powder inhaler, the indicating wheel 42 is rotatably supported to the underside of the divider 14.

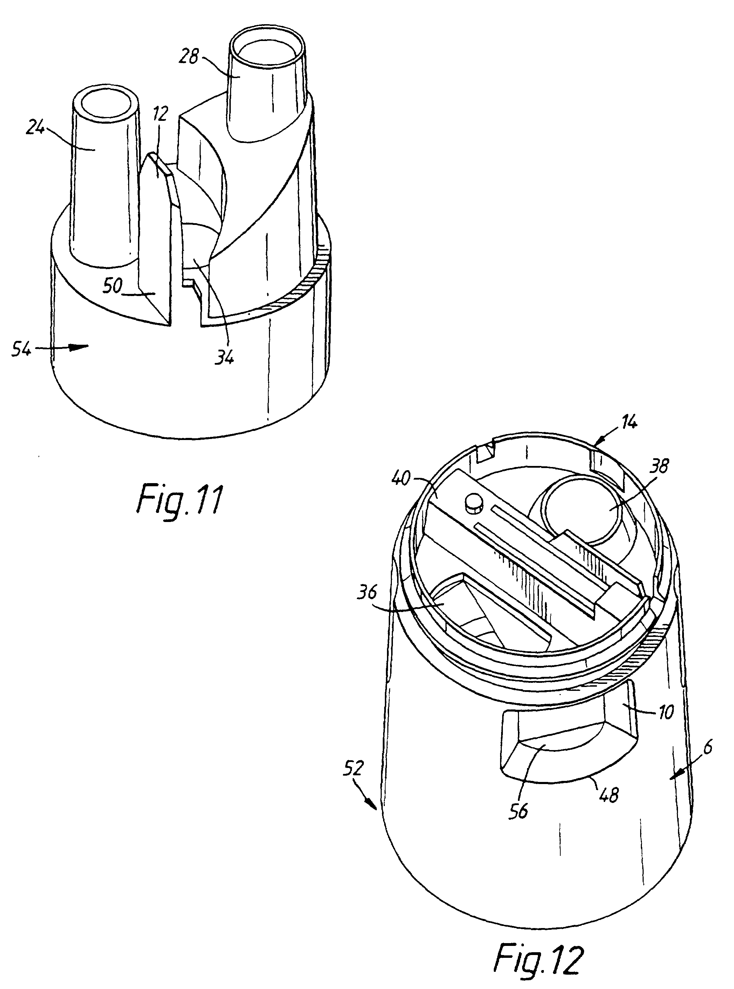

Figure 11 illustrates a structural unit 54 of a powder inhaler in accordance with a fifth embodiment of the present invention.

This inhaler is a modification of the inhaler of the above-described fourth embodiment. This inhaler differs from the inhaler of the above-described fourth embodiment in comprising a structural unit 54 which is a single part moulded from a transparent material and combines both the inhalation unit 22 and the storage unit 26.

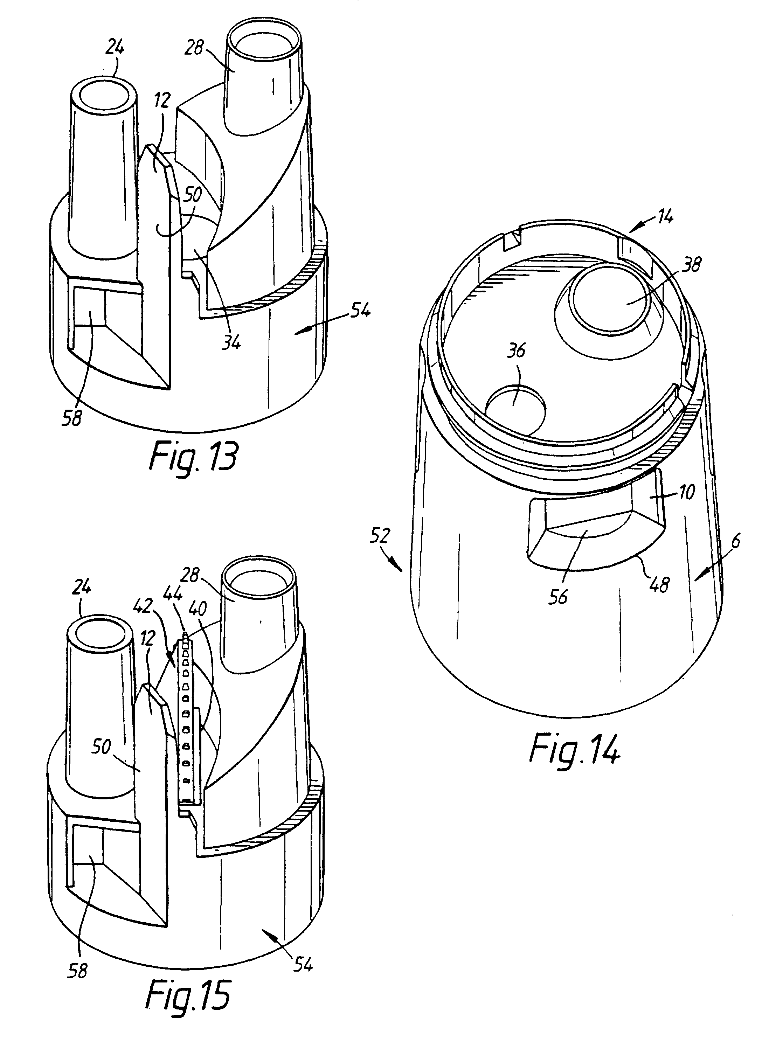

Figures 12 and 13 illustrate respectively a body part 52 and a structural unit 54 or a powder inhaler in accordance with a sixth embodiment of the present invention.

This inhaler is a modification of the inhaler of the above-described fifth embodiment. In this embodiment the body part 52 differs in that a lower section of the recess 48 in the inhaler body 6 is cut away to provide an opening 56 into the inhaler body 6 and in that the lower end of the inhalation channel 24 is provided with a lateral opening 58. During inhalation by a user, air is drawn through the opening 56 in the recess 48 and then the opening 58 in the inhalation channel 24 where a dose of powder is entrained, which powder is then drawn up the inhalation channel 24 into and through the air chamber and out of the outlet nozzle 4 of the mouthpiece 2.

Figures 14 and 15 illustrate respectively a body part 52 and a structural unit 54 of a powder inhaler in accordance with a seventh embodiment of the present invention.

This inhaler is a modification of the inhaler of the above-described sixth embodiment. This inhaler differs from the inhaler of the above-described sixth embodiment in that the structural unit 54 includes the supporting means 40 for rotatably supporting the indicating wheel 42 instead of the divider 14 and in that the divider 14 is formed with a substantially flat top surface. In this way, the risk of powder accumulating at this top surface is minimized. This is of particular importance where the top surface of the divider 14 forms the lower wall of the air chamber of the mouthpiece 2.

In each of the inhalers of the above-described fourth to seventh embodiments the storage chamber 28 is crescent-shaped in plan view and thereby provides an increased storage capacity. It will be understood, however, that the storage chamber 28 may be formed as a cylinder as in the above-described known powder inhaler.

Finally, it will be understood by a person skilled in the art that the present invention is not limited to the described embodiments but can be modified in many different within the scope of the appended claims.

## Claims

1. A powder inhaler for administering powder by inhalation, comprising:
a substantially cylindrical inhaler body (6) having an opening (10) therein:
an inhalation unit (22) disposed in the inhaler body (6), the inhalation unit (22) comprising an inhalation channel (24) through which powder is in use inhaled;
a dosing unit (16) for providing a dose of powder to the inhalation channel (24) disposed in the inhaler body (6) so as to be rotatable about the central axis thereof, wherein the dosing unit (16) comprises a central shaft (20) which is co-axial with the central axis of the inhaler body (6) and has a spiral groove or protrusion (46) on the end face thereof; and
an indicating wheel (42) for providing an indication as to the usage of the inhaler disposed in the inhaler body (6), the indicating wheel (42) having a toothed periphery (44) for engaging the spiral groove or protrusion (46) on the shaft (20) and being disposed such that at least a part thereof is visibie through the opening (10) and so as to be rotatable within a diametrical plane containing the central axis of the inhaler body (6);
**characterized in that** one side surface of the indicating wheel (42) includes at least one indication which is representative of the usage of the inhaler, **in that** the inhaler body (6) includes a recess (48) in which the opening (10) is provided and **in that** the opening (10) has a radial component which allows at least a part of the one side surface of the indicating wheel (42) to be viewed.

2. The inhaler according to claim 1, further comprising a storage unit (26) disposed in the inhaler body (6), the storage unit (26) comprising a storage chamber (28) for storing powder.

3. The inhaler according to claim 2, wherein the storage unit (26) is formed of a transparent material and further comprises a portion (12) which substantially fills the opening (10).

4. The inhaler according to claim 2 or 3, wherein the inhalation unit (22) and the storage unit (26) are formed as a single integral unit.

5. The inhaler according to any of claims 1 to 4, further comprising a divider (14) substantially closing one end of the inhaler body (6).

6. The inhaler according to claim 5, wherein the recess (48) comprises first and second opposing surfaces which are substantially parallel to the major surface of the divider (14) and at least first and second side surfaces joining the first and second opposing surfaces, the opening (10) being formed in one of the side surfaces.

7. The inhaler according to claim 5 or 6, wherein the inhaler body (6) and the divider (14) are formed as a single integral unit.

8. The inhaler according to any of claims 2 to 7, wherein the storage unit (26) further comprises supporting means (40) for rotatably supporting the indicating wheel (42).

9. The inhaler according to any of claims 5 to 7, wherein the divider (14) comprises supporting means (40) for rotatably supporting the indicating wheel (42).

10. The inhaler according to any of claims 1 to 9, wherein the inhaler body (6) further comprises an air inlet (56) in a side wall thereof, the air inlet (56) allowing air to be drawn to the dosing unit (16) and through the inhalation channel (24).

11. The inhaler according to claim 10, wherein the air inlet (56) is provided in the recess (48).

12. The inhaler according to any of claims 1 to 11, wherein the one side surface of the indicating wheel (42) includes numerals indicating the number of doses administered and/or remaining.

13. The inhaler according to any of claims 1 to 12, wherein the one side surface of the indicating wheel (42) includes an at least part circular band of varying width indicating the number of doses administered and/or remaining.

## Patentansprüche

1. Pulverinhalator zur Verabreichung von Pulver durch Inhalierung mit Folgendem:
einem im Wesentlichen zylindrischen Inhalatorkörper (6) mit einer Öffnung (10),
einer im Inhalatorkörper (6) angeordneten Inhalationseinheit (22) mit einem Inhalationskanal (24), durch den das Pulver im Gebrauch inhaliert wird,
einer Dosiereinheit (16) zur Bereitstellung einer Pulverdosis zum Inhalationskanal (24), die so im Inhalatorkörper (6) angeordnet ist, dass sie sich um dessen mittlere Achse drehen kann, wobei die Dosiereinheit (16) eine mittlere Welle (20) umfasst, die mit der mittleren Achse des Inhalatorkörpers (6) koaxial ist und an ihrer Endfläche eine spiralförmige Nut bzw. einen spiralförmigen Vorsprung (46) aufweist, und
einem Angaberad (42) zur Bereitstellung einer Angabe über den Gebrauch des im Inhalatorkörper (6) angeordneten Inhalators, wobei das Angaberad (42) eine gezahnte Peripherie (44) zur Ineingriffnahme der spiralförmigen Nut bzw. des spiralförmigen Vorsprungs (46) auf der Welle (20) hat und so angeordnet ist, dass mindestens ein Teil davon durch die Öffnung (10) sichtbar ist und es in einer die mittlere Achse des Inhalatorkörpers (6) enthaltenden diametralen Ebene drehbar ist,
**dadurch gekennzeichnet, dass** eine Seitenfläche des Angaberads (42) mindestens eine Angabe enthält, die für den Gebrauch des Inhalators repräsentativ ist, dass der Inhalatorkörper (6) eine Ausnehmung (48) enthält, in der die Öffnung (10) vorgesehen ist, und dass die Öffnung (10) eine radiale Komponente hat, dank derer mindestens ein Teil der einen Seitenfläche des Angaberads (42) eingesehen werden kann.

2. Inhalator nach Anspruch 1, weiterhin mit einer im Inhalatorkörper (6) angeordneten Vorratseinheit (26) mit einer Vorratskammer (28) zum Lagern von Pulver.

3. Inhalator nach Anspruch 2, bei dem die Vorratseinheit (26) aus einem transparenten Material gebildet ist und weiterhin einen Abschnitt (12) umfasst, der die Öffnung (10) im Wesentlichen füllt.

4. Inhalator nach Anspruch 2 oder 3, bei dem die Inhalationseinheit (22) und die Vorratseinheit (26) als eine einzige integrale Einheit gebildet sind.

5. Inhalator nach einem der Ansprüche 1 bis 4, weiterhin mit einem Teiler (14), der ein Ende des Inhalatorkörpers (6) im Wesentlichen schließt.

6. Inhalator nach Anspruch 5, bei dem die Ausnehmung (48) Folgendes umfasst: eine erste und eine zweite Fläche, die sich gegenüberliegen und im Wesentlichen parallel zu der Hauptfläche des Teilers (14) verlaufen, und mindestens eine erste und eine zweite Seitenfläche, die an die erste und zweite Fläche, die sich gegenüberliegen, angrenzen, wobei die Öffnung (10) in einer der Seitenflächen gebildet ist.

7. Inhalator nach Anspruch 5 oder 6, bei dem der Inhalatorkörper (6) und der Teiler (14) als eine einzige integrale Einheit ausgebildet sind.

8. Inhalator nach einem der Ansprüche 2 bis 7, bei dem die Vorratseinheit (26) weiterhin ein Stützmittel (40) zum drehbaren Stützen des Angaberads (42) umfasst.

9. Inhalator nach einem der Ansprüche 5 bis 7, bei dem der Teiler (14) ein Stützmittel (40) zum drehbaren Stützen des Angaberads (42) umfasst.

10. Inhalator nach einem der Ansprüche 1 bis 9, bei dem der Inhalatorkörper (6) weiterhin in einer seiner Seitenwände einen Lufteinlass (56) umfasst, dank dessen Luft zur Dosiereinheit (16) und durch den Inhalationskanal (24) gezogen werden kann.

11. Inhalator nach Anspruch 10, bei dem der Lufteinlass (56) in der Ausnehmung (48) vorgesehen ist.

12. Inhalator nach einem der Ansprüche 1 bis 11, bei dem die eine Seitenfläche des Angaberads (42) Ziffern aufweist, die angeben, wie viele Dosen verabreicht worden sind und/oder noch übrig sind.

13. Inhalator nach einem der Ansprüche 1 bis 12, bei dem die eine Seitenfläche des Angaberads (42) ein mindestens teilweise kreisförmiges Band unterschiedlicher Breite aufweist, das angibt, wie viele Dosen verabreicht worden sind und/oder noch übrig sind.

## Revendications

1. Inhalateur à poudre pour l'administration de poudre par inhalation, comprenant :
un corps d'inhalateur essentiellement cylindrique (6) doté d'une ouverture (10) ;
une unité d'inhalation (22) disposée dans le corps d'inhalateur (6), l'unité d'inhalation (22) comprenant un canal d'inhalation (24) à travers lequel la poudre est inhalée lors de l'utilisation ;
une unité de dosage (16) pour fournir une dose de poudre au canal d'inhalation (24), disposée dans le corps d'inhalateur (6) de manière à être rotative autour de son axe central,
dans lequel l'unité de dosage (16) comprend un fût central (20) coaxial avec l'axe central du corps d'inhalateur (6) et possède un sillon ou une saillie en spirale (46) sur sa face d'extrémité ; et
une roue indicatrice (42) pour fournir une indication de l'usage de l'inhalateur, disposée dans le corps d'inhalateur (6), la roue indicatrice (42) présentant une périphérique dentée (44) pour venir en prise avec le sillon ou la saillie en spirale (46) sur le fût (20) et étant disposée de telle sorte qu'une partie au moins de celle-ci soit visible à travers l'ouverture (10) et de manière à être rotative dans un plan diamétral contenant l'axe central du corps d'inhalateur (6) ;
**caractérisé en ce qu'**une surface latérale de la roue indicatrice (42) comporte au moins une indication reflétant l'usage de l'inhalateur, **en ce que** le corps d'inhalateur (6) comporte un renfoncement (48) dans lequel l'ouverture (10) est ménagée, et **en ce que** l'ouverture (10) présente une composante radiale permettant de voir au moins une partie de ladite surface latérale de la roue indicatrice (42).

2. Inhalateur selon la revendication 1, comprenant en outre une unité de stockage (26) disposée dans le corps d'inhalateur (6), l'unité de stockage (26) comprenant une chambre de stockage (28) pour stocker la poudre.

3. Inhalateur selon la revendication 2, dans lequel l'unité de stockage (26) est formée d'une matière transparente et comprend en outre une portion (12) qui remplit essentiellement l'ouverture (10).

4. Inhalateur selon la revendication 2 ou 3, dans lequel l'unité d'inhalation (22) et l'unité de stockage (26) prennent la forme d'une seule unité monobloc.

5. Inhalateur selon l'une quelconque des revendications 1 à 4, comprenant en outre un séparateur (14) obturant essentiellement une extrémité du corps d'inhalateur (6).

6. Inhalateur selon la revendication 5, dans lequel le renfoncement (48) comprend des première et deuxième surfaces opposées essentiellement parallèles à la surface principale du séparateur (14) et au moins des première et deuxième surfaces latérales joignant les première et deuxième surfaces opposées, l'ouverture (10) étant formée dans une des surfaces latérales.

7. Inhalateur selon la revendication 5 ou 6, dans lequel le corps d'inhalateur (6) et le séparateur (14) prennent la forme d'une seule unité monobloc.

8. Inhalateur selon l'une quelconque des revendications 2 à 7, dans lequel l'unité de stockage (26) comprend en outre un moyen de support (40) pour supporter à rotation la roue indicatrice (42).

9. Inhalateur selon l'une quelconque des revendications 5 à 7, dans lequel le séparateur (14) comprend un moyen de support (40) pour supporter à rotation la roue indicatrice (42).

10. Inhalateur selon l'une quelconque des revendications 1 à 9, dans lequel le corps d'inhalateur (6) comprend en outre une admission d'air (56) dans une paroi latérale de celui-ci, l'admission d'air (56) permettant l'aspiration d'air jusqu'à l'unité de dosage (16) et à travers le canal d'inhalation (24).

11. Inhalateur selon la revendication 10, dans lequel l'admission d'air (56) est prévue dans le renfoncement (48).

12. Inhalateur selon l'une quelconque des revendications 1 à 11, dans lequel ladite surface latérale de la roue indicatrice (42) comporte des numéros indiquant le nombre de doses administrées et/ou restantes.

13. Inhalateur selon l'une quelconque des revendications 1 à 12, dans lequel ladite surface latérale de la roue indicatrice (42) comporte une bande au moins partiellement circulaire de largeur variable indiquant le nombre de doses administrées et/ou restantes.
